# EUROPEAN PATENT SPECIFICATION

(11) **EP 1 493 743 B1**
(45) Date of publication and mention of the grant of the patent: **03.09.2008**
(21) Application number: 04020599.9
(22) Date of filing: 05.04.2001
(51) Int. Cl.: C07D 401/14, C07D 405/14, C07D 409/14, C07D 413/14, C07D 417/14, C07D 471/04, C07D 211/58

(54) **Substituted bipiperidine intermediates and derivatives thereof**
Substituierte Bipiperidine -Zwischenverbindungen und deren Derivate
Des bipéridines substitueés en tant qu'intermédiares et leurs dérivés

(30) Priority: 08.04.2000 GB 0008626; 03.08.2000 GB 0019111; 11.10.2000 SE 0003664
(43) Date of publication of application: 05.01.2005
(62) Divisional of application: 01920053.4
(73) Proprietor: AstraZeneca AB, 151 85 Södertälje (SE)
(72) Inventor: Springthorpe, Brian, Loughborough Leics. LE11 5RH (GB); Sanganee, Hitesh, Loughborough Leics. LE11 5RH (GB); Rigby, Aaron, Loughborough Leics. LE11 5RH (GB); Lawrence, Louise, Coalville, Leicestershire LE67 4PB (GB)

(56) References cited:
- WO-A-98/05292

## Description

The present application is a divisional of European Patent Application No. 01920053.4, now granted as EP-B-1274701.

The present invention concerns N-protected and N-unsubstituted [1,4']bipiperidine derivatives (intermediates) that are useful in the preparation of piperidine derivatives having pharmaceutical activity (for example as modulators of chemokine receptor (such as CCR3) activity, or as antagonists of H1). WO98/05292 discloses certain [1,4']bipiperidine derivatives.

European Patent Application No. 01920053.4 discloses pharmaceutically active compounds of formula (I): 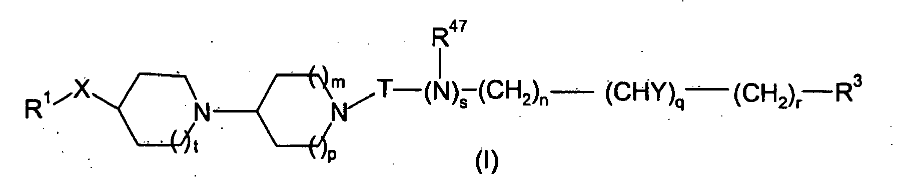 wherein:
q, s and t are, independently, 0 or 1;
n and r are, independently, 0, 1, 2, 3, 4 or 5;
m and p are, independently, 0, 1 or 2;
X is CH_{2;} C(O), O, S, S(O), S(O)₂ or NR³⁷; provided that when m and p are both l then X is not CH₂;
Y is NHR² or OH;
T is C(O), C(S), S(O)₂ or CH₂;
R¹ is hydrogen, C₁₋₆ alkyl, aryl or heterocyclyl;
R² and R¹⁷ are, independently, hydrogen, C₁₋₆ alkyl, aryl(C₁₋₄)alkyl or CO(C₁₋₆ alkyl);
R³ is C₁₋₆ alkyl {optionally substituted by halogen, CO₂R⁴ or phthalimide}, CR^{3a}R^{3b}R^{3c},
C₂₋₄ alkenyl {optionally substituted by aryl or heterocyclyl}, C₃₋₇ cycloalkyl (optionally substituted by C₁₋₄ alkyl, aryl or oxo}, C₃₋₇ cycloalkenyl {optionally substituted by oxo, C₁-₆ alkyl or aryl}, aryl, heterocyclyl, thioaryl or thioheterocyclyl;
R^{3a} is hydrogen, C₁₋₆ alkyl, C₁₋₆ alkoxy or C₃₋₇ cycloalkyl; R^{3b} is aryl, heterocyclyl, S(O)₂aryl or S(O)₂heterocyclyl; and R^{3c} is C₁₋₆ alkyl, C₁₋₄ haloalkyl, hydroxy,
heterocyclyl(C₁₋₄ alkyl) or aryl;
   wherein, unless stated otherwise, the foregoing aryl and heterocyclyl moieties are optionally substituted by: halogen, OH, SH, NO₂, oxo, C₁₋₆ alkyl {itself optionally substituted by halogen, OC(O)C₁₋₆ alkyl, S(O)₂R⁴⁸, phenyl (itself optionally substituted by halogen (such as one or two chlorine or fluorine atoms), C₁₋₆ alkyl, S(O)₂R³⁸ or C(O)NR³⁹R⁴⁰), naphthyloxy (itself optionally substituted by halo or C₂₋₆ alkenyl), C₃₋₁₀ cycloalkyl (itself optionally substituted by C₁₋₄ alkyl or oxo) or NR⁴¹C(O)OCH₂(fluoren-9-yl)}, NR⁴¹C(O)OCH₂(fluoren-9-yl), C₁₋₆ alkoxy {itself optionally substituted by halogen, C₁₋₆ alkoxy, NHCO₂(C₁₋₆ alkyl), CO₂R⁴, NR⁵R⁶ or phenyl (itself optionally substituted by halogen or NO₂)}, C₁₋₆ alkylthio, C₁₋₆ haloalkylthio, C₃₋₁₀ cycloalkyl, NR⁷R⁸, NR⁹C(O)R¹⁰, CO₂R¹¹, C(O)NR¹²R¹³, C(O)R¹⁴, S(O)_{d}R¹⁵, S(O)₂NR⁴²R⁴³, NR⁴⁴S(O)₂R⁴⁵, phenyl {itself optionally substituted by halogen, C₁₋₆ alkyl, C₁₋₆ haloalkyl, CN, NO₂ C₁₋₆ alkoxy (itself optionally substituted by halogen, OH or pyridinyl), phenyl (itself optionally substituted by halogen, C₁₋₆ alkyl, C₁₋₆ haloalkyl, CN, NO₂ C₁₋₆ alkoxy or C₁₋₆ haloalkoxy) or heterocyclyl (itself optionally substituted by halogen, C₁₋₆ alkyl, C₁₋₆ haloalkyl, CN, NO₂, C₁₋₆ alkoxy or C₁₋₆ haloalkoxy)}, heterocyclyl {itself optionally substituted by halogen, C₁. ₆ alkyl, C₁₋₆ haloalkyl, CN, NO₂, C₁₋₆ alkoxy, C₁₋₆ haloalkoxy, phenyl (itself optionally substituted by halogen, C₁₋₆ alkyl, C₁₋₆ haloalkyl, CN, NO₂, C₁₋₆ alkoxy or C₁₋₆ haloalkoxy) or heterocyclyl (itself optionally substituted by halogen, C₁₋₆ alkyl, C₁₋₆ haloalkyl, CN, NO₂, C₁₋₆ alkoxy or C₁₋₆ haloalkoxy)}, phenoxy {itself optionally substituted by halogen, C₁₋₆ alkyl, C₁₋₆ haloalkyl, CN, NO₂, C₁₋₆ alkoxy, C₁₋₆ haloalkoxy, phenyl (itself optionally substituted by halogen, C₁₋₆ alkyl, C₁₋₆ haloalkyl, CN, NO₂, C₁₋₆ alkoxy or C₁₋₆ haloalkoxy) or heterocyclyl (itself optionally substituted by halogen, C₁₋₆ alkyl, C₁₋₆ haloalkyl, CN, NO₂, C₁₋₆ alkoxy or C₁₋₆ haloalkoxy)}, SCN, CN, SO₃H (or an alkali metal salt thereof), methylenedioxy or difluoromethylenedioxy; when aryl is phenyl adjacent substituents may join to form, together with the phenyl ring to which they are attached, a dihydrophenanthrene moiety;
d is 0, 1 or 2;
R⁴ R⁵, R⁶, R⁷, R⁸, R⁹, R¹⁰, R¹¹ R¹², R¹³, R¹⁴, R³⁷, R³⁹, R⁴⁰,R⁴¹, R⁴², R⁴³ and R⁴⁴ are, independently, hydrogen, C₁₋₆ alkyl, aryl (itself optionally substituted by halogen, C₁₋₆ alkyl, C₁₋₆ haloalkyl, CN, NO₂, C₁₋₆ alkoxy or C₁₋₆ haloalkoxy) or heterocyclyl (itself optionally substituted by halogen, C₁₋₆ alkyl, C₁₋₆ haloalkyl, CN, NO₂, C₁₋₆ alkoxy or C₁₋₆ haloalkoxy);
R¹⁵, R³⁸, R⁴⁵ and R⁴⁸ are, independently, C₁₋₆ alkyl (optionally substituted by halogen, hydroxy or C₃₋₁₀ cycloalkyl), C₃₋₆ alkenyl, aryl (itself optionally substituted by halogen, C₁₋₆ alkyl, C₁₋₆ haloalkyl, CN, NO₂, C₁₋₆ alkoxy or C₁₋₆ haloalkoxy) or heterocyclyl (itself optionally substituted by halogen, C₁₋₆ alkyl, C₁₋₆ haloalkyl, CN, NO₂, C₁₋₆ alkoxy or C₁₋₆ haloalkoxy);
or an N-oxide thereof; or a pharmaceutically acceptable salt thereof; or a solvate thereof; provided that:
   when m and p are both 1, n, q and r are all 0, T and X are both S(O)₂, and R¹ is methoxyphenyl then R³ is not propyl; when m, p, q and r are all 1, n is 0, Y is NH₂, T is CO and R¹X is (CH₃)₂N then R³ is not 3,5-dibromo-4-aminophenyl, 1-methylindol-3-yl or 1-(tert-butoxy*carbonyl)indol-3-yl; and when m and p are both 1, n, q and r are all 0, T is CO, X is NH and R¹ is 3-(4-fluorobenzyl)benzimidazol-2-yl then R³ is not 4-fluorophenyl.

European Patent Application No. 01920053.4 also discloses pharmaceutically active compounds of formula (Ia): 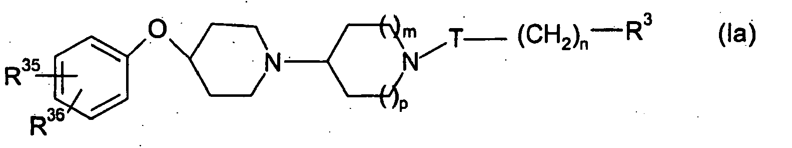 wherein:
T is C(O), C(S), S(O)₂ or CH₂;
n is 0, 1, 2, 3, 4 or 5;
m and p are, independently, 0, 1 or 2 (but are especially both 1);
R³⁵ is hydrogen, cyano, S(O)₂(C₁₋₄ alkyl), S(O)₂(C₁₋₄ haloalkyl), halogen, C₁₋₄ alkyl, C₁₋₄ haloalkyl, C₁₋₄ alkoxy or phenyl (optionally substituted by one or two halogen atoms or by one C(O)NR^{12'}R^{13'}, NR^{9'}C(O)R^{10'}, S(O)₂R^{15'}, S(O)₂NR⁴²R⁴³ or NR⁴⁴S(O)₂R⁴⁵ group);
R³⁶ is hydrogen, halogen or C₁₋₄ alkyl;
R³ is C₁₋₆ alkyl {optionally substituted by halogen, CO₂R⁴ or phthalimide}, C₃₋₇ cycloalkyl {optionally substituted by C₁₋₄ alkyl or oxo}, aryl or heterocyclyl;
   wherein, unless stated otherwise, the foregoing aryl and heterocyclyl moieties are optionally substituted by: halogen, OH, SH, NO₂, oxo, C₁₋₆ alkyl (itself optionally substituted by halogen, OC(O)C₁₋₆ alkyl, phenyl (itself optionally substituted by halo or C₁₋₆ alkyl), naphthyloxy (itself optionally substituted by halo or C₂₋₆ alkenyl) or NR⁴C(O)OCH₂(fluoren-9-yl)), C₁₋₆ alkoxy (itself optionally substituted by halogen, CO₂R⁴, NR⁵R⁶ or phenyl (itself optionally substituted by halogen or NO₂)) C₁₋₆ alkylthio, nitro, C₃₋₇ cycloalkyl, NR⁷R⁸, NR⁹C(O)R¹⁰, CO₂R¹¹, C(O)NR¹²R¹³, C(O)R¹⁴, S(O)₂R¹⁵, phenyl (itself optionally substituted by NO₂ or C₁₋₆ alkoxy (itself optionally substituted by OH or pyridinyl)), phenoxy, SCN, CN, SO₃H (or an alkali metal salt thereof) or methylenedioxy; when aryl is phenyl adjacent substituents may join to form, together with the phenyl ring a dihydrophenanthrene moiety;
R⁴ R⁵, R⁶ R⁷ , R⁸ , R⁹ R^{9'}, R¹⁰, R¹⁰, R¹¹, R¹² , R^{12'} R¹³, R^{13'}, R¹⁴ R⁴² R⁴³ and R⁴⁴ are, independently, hydrogen, C₁₋₆ alkyl or phenyl;
R¹⁵, R¹⁵' and R⁴⁵ are, independently, C₁₋₆ alkyl or phenyl;
   or a pharmaceutically acceptable salt thereof.

The present invention provides a compound of formula (XIIIa): 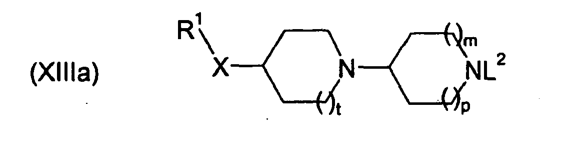 wherein:
L² is hydrogen, tert-butoxycarbonyl or benzyl;
t is 1;
m and p are 1 ;
X is O;
R¹ is either phenyl substituted with one or more of fluorine, chlorine, C₁₋₄ alkyl or C₁₋₄ alkoxy, or R¹ is phenyl substituted by R³⁵ and R³⁶;
R³⁵ is hydrogen, cyano, S(O)₂(C₁₋₄ alkyl), S(O)₂(C₁₋₄ haloalkyl), halogen, C₁₋₄ alkyl, C₁₋₄ haloalkyl, C₁₋₄ alkoxy or phenyl (optionally substituted by one or two halogen atoms or by one C(O)NR^{12'}R^{13'}, NR^{9'}C(O)R^{10'}, S(O)₂R^{15'}, S(O)₂NR⁴²R⁴³ or NR⁴⁴S(O)₂R⁴⁵ group);
R³⁶ is hydrogen, halogen or C₁₋₄ alkyl;
R⁹, R^{10'}, R^{12'}, R^{13'}, R⁴², R⁴³ and R⁴⁴ are, independently, hydrogen, C₁₋₆ alkyl or phenyl; and,
R^{15'} and R⁴⁵ are, independently, C₁₋₆ alkyl or phenyl.

Halogen includes fluorine, chlorine, bromine and iodine.

Alkyl groups and moieties are straight or branched chain and are, for example, methyl, ethyl, n-propyl, iso-propyl or tert-butyl.

In another aspect the present invention provides a compound of formula (XIII) (also referred to as a compound of formula (II) in the processes described below): 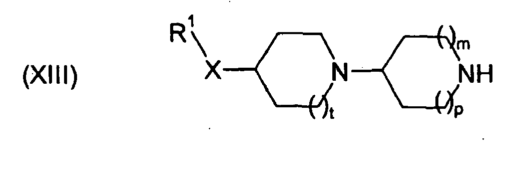 wherein R¹, X, t, p and m are as defined above.

In a further aspect the present invention provides a compound of formula (XIV): 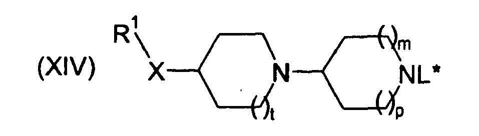 wherein L* is BOC or a benzyl group; and R¹, X, t, p and m are as defined above.

In a still further aspect R¹ is phenyl substituted with one or more of fluorine, chlorine, C₁₋₄ alkyl (especially methyl) or C₁₋₄ alkoxy (especially methoxy); for example R¹ is phenyl substituted by one, two or three of: fluoro, chloro, methyl or methoxy. For example R¹ is 3,4-dichlorophenyl or 3,4-difluorophenyl.

A compound of formula (I), wherein s is 0, can be prepared by coupling a compound of formula (II): 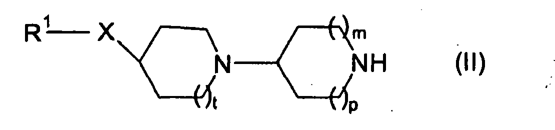 with a compound of formula (III: 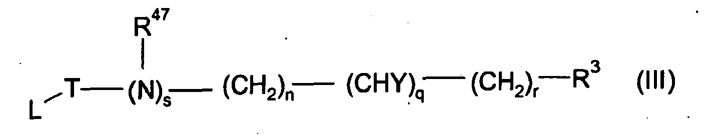 wherein L is a suitable leaving group, and the variables Y and T are optionally protected during the course of the reaction by standard protecting groups known in the art and deprotected in a separate step or during the reaction work-up. For example:
- when T is carbonyl, L can be OH and the coupling can be carried out in the presence of • a coupling agent (such as bromo-tris-pyrrolidino-phosphonium hexafluorophosphate, (known as PYBROPT^{™}), oxalyl chloride, thionyl chloride or N,N'-carbonyl diimidazole, or another coupling agent known to a person skilled in the art); or,
- when T is sulphonyl, L can be chloro and the coupling can be carrier out in the presence of a suitable base (such as potassium carbonate) in a suitable solvent (such as acetone).

A compound of formula (I), wherein s is 1, R⁴⁷ is hydrogen and T is CO, can be prepared by reacting a compound of formula (II), wherein m and p are both 1, with an with an isocyanate O=C=N-(CH₂)ₙ(CH₂)ᵣ-R³.

A compound of formula (II) can be prepared by deprotecting a compound of formula (IV) (which is an example of a compound of formula (XIV)): 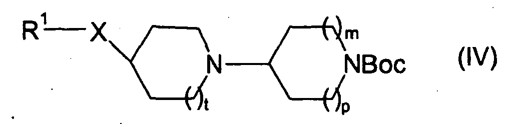 for example using trifluoroacetic acid in a suitable solvent (such as dichloromethane) or using a source of hydrogen chloride in a suitable solvent (such as dioxane).

A compound of formula (IV), wherein X is O, can be prepared by reacting a compound of formula (V): 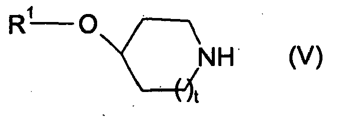 with a compound of formula (VI): 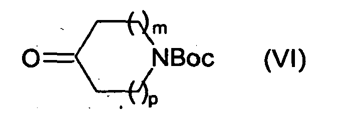 in the presence of NaBH(OAc)₃ and acetic acid.

Alternatively, a compound of formula (1), wherein s, n, q and r are all 0 and T is CO, can be prepared by reacting a compound of formula (XIII): 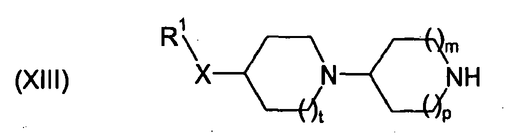 with an acid: R³CO₂H. A compound of formula (XIII) can be prepared by deprotecting a compound of formula (XIV): 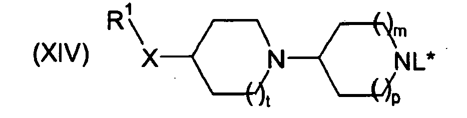 wherein L* is BOC or a benzyl group. A compound of formula (XIV) can be prepared by performing a fluoride displacement reaction on FR¹ in the presence of compound of formula (XV):

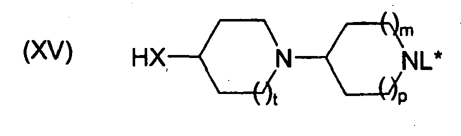

A compound of formula (XV) can be prepared by coupling a compound of formula (XVI) with a compound of formula (XVII):

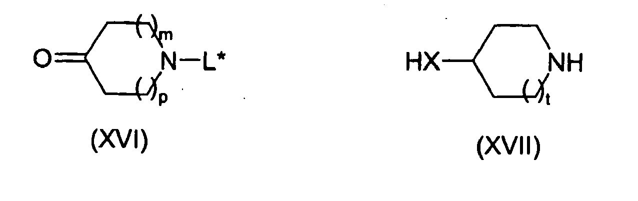

Compounds of formula (V), (VI), (XVI) and (XVII) can be prepared by using or adapting methods described in the art.

The invention will now be illustrated by the following non-limiting Examples. Some Examples illustrate how the compounds of the present invention can be used in the preparation of compounds of formulae (I) or (Ia). In the Method and Example, and unless stated otherwise:
(i) when given, ¹H NMR data is quoted and is in the form of delta values for major diagnostic protons, given in parts per million (ppm) relative to tetramethylsilane (TMS) as an internal standard, determined at 300MHz or 400MHz using perdeuterio DMSO-D6 (CD₃SOCD₃) or CDCl₃ as the solvent unless otherwise stated;
(ii) mass spectra (MS) were run with an electron energy of 70 electron volts in the chemical ionisation (CI) mode using a direct exposure probe; where indicated ionisation was effected by electron impact (EI) or fast atom bombardment (FAB); where values for m/z are given, generally only ions which indicate the parent mass are reported, and unless otherwise stated the mass ion quoted is the positive mass ion - (M+H)⁺;
(iii) the title and sub-titled compounds of the examples and methods were named using the AUTONOM program from Beilstein informationssysteme GmbH;
(iv) unless stated otherwise, reverse phase HPLC was conducted using a Symmetry, NovaPak or Ex-Terra reverse phase silica column; and
(v) the following abbreviations are used:

| | | | | |
|---|---|---|---|---|
| MTBE | tert-butyl methyl ether | | THF | tetrahydrofuran |
| DMF | N,N-dimethylformamide | | DCM | dichloromethane |
| Boc or BOC | tert-butoxycarbonyl | | DMSO | dimethylsulfoxide |
| HPLC | high pressure liquid chromatography | | Ac | Acetate |
| PYBROP^{™} | bromo-tris-pyrrolidino-phosphonium hexafluorophosphate | | aq | aqueous |

### Method 1

This Method illustrates the preparation of 4-(3,4-dichlorophenoxy)piperidine. Step a: *tert*-Butyl4-(3,4-dichlorophenoxy)-1-piperidinecarboxylate

Diethyl azodicarboxylate (41.0ml) was added to a solution of triphenylphosphine (62.9g) in tetrahydrofuran (800ml) at 0°C. After 15 minutes 3,4-dichlorophenol (39.1g) was added, after a further 15 minutes tert-butyl 4-hydroxy-1-piperidinecarboxylate (48.3g) in tetrahydrofuran (400ml) was added dropwise over 30 min. The solution was stirred at room temperature for 16 hours and concentrated to a small volume. Purification by chromatography (ethyl acetate : *iso*-hexane 95:5) gave the sub-title compound as an oil (61.3g).
MS: APCI(+ve): 246 (M-BOC+2H)

### Step b: 4-(3,4-Dichlorophenoxy)piperidine

The product from Step a was dissolved in dichloromethane (600ml) and trifluoroacetic acid (300ml) was added. After 24 hours at room temperature the solution was evaporated and the resultant gum triturated under ether to give the sub-titled product as a solid (36.6g). The free base was liberated by addition of aqueous NaOH (2M) and extraction with ethyl acetate followed by evaporation of solvent to give the title compound as a gum (25g).

¹H NMR: δ(CDCl₃) 1.77 (1H, br s), 2.05-2.26 (4H, m), 3.20-3.49 (4H, m), 4.61 (1H, s), 6.69-7.52 (3H, m).

### Example 1

This Example illustrates the preparation of 4-(3,4-Dichloro-phenoxy)-[1,4']bipiperidinyl-1'-carboxylic acid *tert-*butyl ester, 4-(3,4-Dichloro-phenoxy)-[1,4']bipiperidine, and [4-(3,4-dichloro-phenoxy)-[1,4']bipiperidinyl-1'-yl]-(3-methanesulfonyl-phenyl)-methanone acetate.

### Step a: 4-(3,4-Dichloro-phenoxy)-[1,4']bipiperidinyl-1'-carboxylic acid tert-butyl ester

4-(3,4-Dichlorophenoxy)piperidine (Method 1; 1.5g) was dissolved in 1,2-dichloroethane (21ml). 1-Boc-4-piperidone was added (1.21g) followed by NaBH(OAc)₃ (1.81g) and acetic acid (0.37g). After 18 hours at room temperature aqueous NaOH (1M) solution and diethyl ether were added. The product was extracted with diethyl ether, the combined organic extracts dried with MgSO₄ and concentrated. Purification by silica chromatography (dichloromethane : methanol 92:8) gave the sub-title product (1.97g).
MS: APCI(+ve): 429 (M+H)

### Step b: 4-(3,4-Dichloro-phenoxy)-[1,4']bipiperidine

The product of Step a was dissolved in dichloromethane (30ml) and trifluoroacetic acid (15ml) was added. After 4 hours at room temperature the solution was evaporated and the resultant gum triturated under ether to give the trifluoroacetate salt of the sub-titled product as a solid (1.15g). The free base was liberated by addition of aqueous NaOH (2M) and extraction with ethyl acetate followed by evaporation of solvent to give the sub-title compound as a solid (0.68g).

¹H NMR: δ(CDCl₃) 1.38-1.51 (2H, m), 1.74-2.02 (6H, m), 2.38-2.50 (3H, m), 2.56-2.61 (2H, m), 2.79-2.86 (2H, m), 3.14-3.18 (2H ,m), 4.22-4.28 (1H, m), 6.73-7.32 (3H, m).

### Step c: [4-(3,4-Dichloro-phenoxy)-[1,4']bipiperidinyl-1'-yl]-(3-methanesulfonyl-phenyl)-methanone acetate

The product of Step b (0.15g) was dissolved in THF (4ml), bromo-tris-pyrrolidino-phosphonium hexafluorophosphate (PYBROP^{™}; 0.235g), 3-methylsulphonylbenzoic acid (0.091g) and N,N-di-*iso*-propylethylamine (0.238ml) were added. After 18 hours at room temperature ethyl acetate and aqueous NaHCO₃ solution were added. The product was extracted with ethyl acetate, the combined organic extracts dried with Na₂SO₄ and concentrated. Purification by reverse phase HPLC (with a gradient eluent system (45% MeCN/NH₄OAc_{aq} (0.1%) to 95% MeCN//NH₄OAc_{aq} (0.1%)) gave the title compound (0.095g).

¹H NMR: δ( DMSO-D6) 1.44-1.94 (8H, m), 2.37-2.77 (5H, m), 3.07-3.55 (6H, m), 4.40 (1H, m), 4.50-4.53 (1H, m), 6.96-8.02 (7H, m).

Melting point: 60-61°C becomes a gum.

Melting point of free base: 154°C.

### Example 2

This Example illustrates the preparation of 4-(3,4-Difluoro-phenoxy)-[1,4']bipiperidinyl-1'-carboxylic acid tert-butyl ester, 4-(3,4-Difluoro-phenoxy)-[1,4']bipiperidinyl, and (4-amino-3-methoxy-phenyl)-[4-(3,4-difluoro-phenoxy)-[1,4']bipiperidinyl-1'-yl]-methanone.

### Step a: 4-(3,4-Difluoro-phenoxy)-[1,4']bipiperidinyl-1'-carboxylic acid tert-butyl ester

This compound was prepared by the method of Example 1, Step a using 4-(3,4-difluorophenoxy)piperidine to give the sub-title compound as a solid (0.48g).
MS: APCI(+ve): 397 (M+H)

### Step b: 4-(3,4-Difluoro-phenoxy)-[1,4']bipiperidinyl

This compound was prepared by the method of Example 1, Step b to give the sub-title compound as a solid (0.36g).
MS: APCI(+ve): 297 (M+H)

### Step c: (4-Amino-3-methoxy-phenyl)-[4-(3,4-difluoro-phenoxy)-[1,4']bipiperidinyl-1'-yl]-methanone

This compound was prepared by the method of Example 1, Step c using 4-amino-3-methoxybenzoic acid to give the title compound as a gum (0.133g).

¹H NMR: δ(CDCl₃) 1.50-1.60 (2H, m), 1.85-1.93 (4H, m), 2.04-2.08 (2H, m), 2.58-2.62 (2H, m), 2.69-2.75 (1H, m), 2.86-2.90 (4H, m), 3.86 (3H, s), 3.86 (2H, m), 4.25-4.30 (1H, m), 6.50-6.61 (1H, m), 6.65 (1H, dd), 6.70-6.75 (1H. m), 6.85 (1H, dt), 6.94 (1H, s), 7.01-7.09 (1H, m).

### Example 3

This Example illustrates the preparation of 4-(3,4-dichlorophenoxy)-[1,4']bipiperidinyl-1'-carboxylic acid tert-butyl ester, 4-(3,4-dichlorophenoxy)-[1,4']bipiperidine, and [4-(3,4-dichloro-phenoxy)-[1,4']bipiperidinyl-1'-yl]-(2-methanesulfonyl-phenyl)-methanone.

### Step 1: Preparation of 4-hydroxy-[1,4']bipiperidinyl-1'-carboxylic acid tert-butyl ester

To 1-tert-butoxycarbonyl-4-piperidone (200g, 1.01mol) in tetrahydrofuran (THF) (1500ml) was added 4-hydroxypiperidine (78.1 g, 0.77mol). The resultant slurry was stirred for 30 minutes before cooling the reaction mixture with ice/water, acetic acid (47ml) is then added (exotherm) which caused precipitation. The slurry was allowed to warm to room temperature before the addition of sodium triacetoxyborohydride (236g, 1.12mol) which was washed in with THF (500ml). The resultant slurry was stirred overnight at room temperature. To the reaction mixture was added water (2000ml) to give a solution. The solution was then extracted with diethyl ether (3 x 1800ml). The aqueous phase was basified with 10% aq NaOH (950ml) and extracted with dichloromethane (DCM) (3 x 1500ml). The combined DCM layers are died (MgSO₄), filtered and the solvent removed to give the sub-titled compound as a yellow viscous oil, (177g, 81%; MS: (M+H)285).

### Step 2: Preparation of 4-(3,4-dichlorophenoxy)-[1,4']bipiperidinyl-1'-carboxylic acid tert-butyl ester

To a solution of potassium tert-butoxide (139.0g, 1.24mol) in THF (500ml) was added a solution of the product of Step 1 (176.2g, 0.62mol) in THF (1000ml). The reaction mixture was stirred 10 minutes before the additon of 3,4 dichlorofluorobenzene (122.8g, 0.74mol), this caused a green colouration that subsequently faded. The reaction mixture was then heated at reflux for 90 minutes. The reaction mixture was then cooled to room temperature before the addition of saturated NaHCO₃ (1600ml). The layers were separated and the organic layer stripped to leave an orange semi-solid. The solid was dissolved in DCM (1500ml) and dried (MgSO₄), filtered and the solvent removed. To the resultant solid was added methyl tert-butyl ether (MTBE) (54ml) and iso-hexane (1000ml) to give a slurry which was stirred overnight. The slurry was then filtered and washed with isohexane (200ml) and the solid dried *in vacuo* at 50°C to give the sub-titled compound as a pale powder, (211.6g, 80%; MS: (M+H) 429).

### Step 3: Preparation of 4-(3,4-dichlorophenoxy)-[1,4']bipiperidine

The product of Step 2 (10.15g, 23.6mmol) was dissolved in dichloromethane (150ml) and trifluoroacetic acid (40ml, 519mmol) added and the resultant solution stirred. After 90 minutes the dichloromethane and trifluoroacetic acid were removed on a rotary evaporator. The resultant oil was partitioned between ethyl acetate (100ml) and 2M aq - NaOH (100ml). The layers were separated and the organics extracted with 10% aq citric acid (100ml). The layers were separated and the aqueous basified with 2M aq NaOH and extracted with ethyl acetate (200ml). The organics were dried (MgSO₄), filtered and the solvent removed to give the sub-titled product as a pale oil which solidified on standing (4.62g, 59%; MS: (M+H) 329).

### Step 4: Preparation of [4-(3,4-dichloro-phenoxy)-[1,4']bipiperidinyl-1'-yl]-(2-methanesulfonyl-phenyl)-methanone

Oxalyl chloride (55ml, 0.63mol) was added dropwise over 10 minutes to a stirred suspension of 2-methanesulfonyl-benzoic acid (7.1g, 0.036) in DCM (550ml) containing DMF (0.5ml). The solution was then stirred for 2 hours at room temperature. The solution was then evaporated to give a solid that was redissolved in dichloromethane and again evaporated to give a yellow solid. The solid acid chloride was dissolved in DCM (275ml) and was added over 10 minutes to a stirred solution of the product of Step 3(11.0g, 0.033mol) and triethylamine (15.4ml, 0,11mol) in dichloromethane (125ml). The resultant solution was stirred at room temperature for 16 hours. The solution was then washed with water (500ml), 1M aq NaOH (500ml) and water (2 x 500ml). The organic phase was dried (MgSO₄), filterered and the solvent removed to give a pale yellow foam. The foam was triturated with diethyl ether to give the title compound (12.96g, 76%).

Melting point 141°C.

¹H NMR: (400 MHz, CDCl₃) δ 1.39 - 1.63 (1H, m), 1.72 - 2.04 (6H, m), 2.42 - 2.68 (2H, m), 2.73 - 2.92 (3H, m), 3.00 - 3.08 (1H, m), 3.23 (1H, s), 3.28 (2H, s), 3.34 - 3.40 (1H, m), 3.46 - 3.52 (1H, m), 4.21 - 4.30 (1H, m), 4.62 - 4.68 (1H, m), 4.80 - 4.86 (1H, m), 6.72 - 6.76 (1H, in), 6.97 - 7.00 (1H, m), 7.28 - 7.32 (1H, m), 7.32-7.37 (1H, m), 7.56 - 7.61 (1H, m), 7.64 - 7.70 (1H, m), 8.05 - 8.10 (1H, m).

### Example 4

This Example illustrates the preparation of 4-(4-methanesulfonyl-phenoxy)-[1,4']bipiperidinyl-1'-carboxylic acid tert-butyl ester, and 4-(4-methanesulfonyl-phenoxy)-[1,4']bipiperidinyl

### Step a: 4-(4-methanesulfonyl-phenoxy)-[1,4']bipiperidinyl-1'-carboxylic acid tert-butyl ester

To a solution of 4-(4-methanesulfonyl-phenoxy)-piperidine (0.7g) dissolved in THF (5ml) and 1,2-dichloroethane (10ml) with 1-Boc-4-piperidone (0.71g) was added NaBH(OAc)₃ (0.926g) and acetic acid (0.18g). After 16hours at RT aqueous NaOH (1M) solution and dichloromethane were added and the mixture was extracted with dichloromethane. The combined organic extracts were washed with water, dried with magnesium sulfate and concentrated to leave a residue which was purified by chromatography (dichloromethane : methanol 90:10) to give the sub-title product (1.1g; MS: APCI⁺(M+H) 439).

### Step b: 4-(4-methanesulfonyl-phenoxy)-[1,4']bipiperidinyl

The product of step a was dissolved in dichloromethane (20ml) and trifluoroacetic acid (5ml) was added. After 16hours at room temperature the solution was evaporated to leave the title compound as a TFA salt. The free base (0.7g; oil; MS: APCI+(M+H) 339) was liberated by addition of aqueous NaOH (1M) and extraction with dichloromethane followed by evaporation of the solvent.

## Claims

1. A compound of formula (XIIIa): 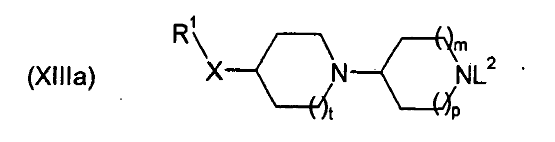 wherein:
L² is hydrogen, tert-butoxycarbonyl or benzyl;
t is 1;
m and p are 1;
X is O;
R¹ is either phenyl substituted with one or more of fluorine, chlorine, C₁₋₄ alkyl or
C₁₋₄ alkoxy, or R¹ is phenyl substituted by R³⁵ and R³⁶;
R³⁵ is hydrogen, cyano, S(O)₂(C₁₋₄ alkyl), S(O)₂(C₁₋₄ haloalkyl), halogen, C₁₋₄ alkyl, C₁₋₄ haloalkyl, C₁₋₄ alkoxy or phenyl (optionally substituted by one or two halogen atoms or by one C(O)NR^{12'}R^{13'}, NR^{9'}C(O)R^{10'}, S(O)₂R^{15'}, S(O)₂NR⁴²R⁴³ or
NR⁴⁴S(O)₂R⁴⁵ group);
R³⁶ is hydrogen, halogen or C₁₋₄ alkyl;
R^{9'} R¹⁰, R^{12'} R^{13'} R⁴², R⁴³ and R⁴⁴ are, independently, hydrogen, C₁₋₆ alkyl or phenyl; and,
R^{15'} and R⁴⁵ are, independently, C₁₋₆ alkyl or phenyl.

2. A compound as claimed in claim 1 wherein R¹ is phenyl substituted with one or more of fluorine, chlorine, C₁₋₄ alkyl or C₁₋₄ alkoxy.

3. A compound as claimed in claim 1 or 2 wherein R¹ is phenyl substituted by one, two or three of fluoro, chloro, methyl or methoxy.

4. A compound as claimed in claim 1 wherein R¹ is 3,4-dichlorophenyl or 3,4-difluorophenyl.

5. A compound as claimed in claim 1 wherein R¹ is 3,4-dichlorophenyl.

6. A compound of formula (XIII): 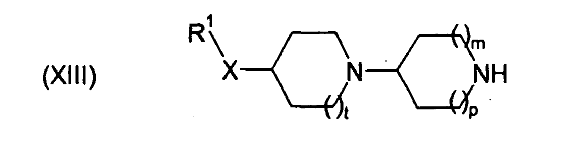 wherein:
t is 1;
m and p are 1;
X is O;
R¹ is either phenyl substituted with one or more of fluorine, chlorine, C₁₋₄ alkyl or
C₁₋₄ alkoxy, or R¹ is phenyl substituted by R³⁵ and R³⁶;
R³⁵ is hydrogen, cyano, S(O)₂(C₁₋₄ alkyl), S(O)₂(C₁₋₄ haloalkyl), halogen, C₁₋₄ alkyl, C₁₋₄ haloalkyl, C₁₋₄alkoxy or phenyl (optionally substituted by one or two halogen atoms or by one C(O)NR^{12'}R¹³', NR^{9'}C(O)R^{10'}, S(O)₂R^{15'}, S(O)₂NR⁴²R⁴³ or
NR⁴⁴S(O)R⁴⁵ group);
R³⁶ is hydrogen, halogen or C₁₋₄ alkyl;
R^{9'}, R^{10'} R^{12'}R^{13'} , R⁴², R⁴³ and R⁴⁴ are, independently, hydrogen, C₁₋₆ alkyl or phenyl; and,
R^{15'} and R⁴⁵ are, independently, C₁₋₆ alkyl or phenyl.

7. A compound as claimed in claim 6 wherein R¹ is phenyl substituted with one or more of fluorine, chlorine, C₁₋₄ alkyl or C₁₋₄ alkoxy.

8. A compound as claimed in claim 7 wherein R¹ is phenyl substituted by one, two or three of: fluoro, chloro, methyl or methoxy.

9. A compound as claimed in claim 6 wherein R¹ is 3,4-dichlorophenyl or 3,4-difluorophenyl.

10. A compound as claimed in claim 6 wherein R¹ is 3,4-dichlorophenyl.

11. A compound of formula (XIV): 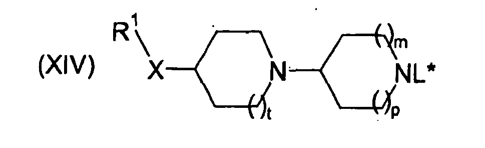 wherein:
L* is BOC or a benzyl group.
t is 1;
m and p are 1;
X is O;
R¹ is either phenyl substituted with one or more of fluorine, chlorine, C₁₄ alkyl or
C₁₋₄ alkoxy, or R¹ is phenyl substituted by R³⁵ and R³⁶;
R³⁵ is hydrogen, cyano, S(O)₂(C₁₋₄ alkyl), S(O)₂(C₁₋₄ haloalkyl), halogen, C₁₋₄ alkyl, C₁₋₄ haloalkyl, C₁₋₄ alkoxy or phenyl (optionally substituted by one or two halogen atoms or by one C(O)NR^{12'}R^{13'}, NR^{9'}C(O)R¹⁰, S(O)₂R^{15'}, S(O)₂NR⁴²R⁴³ or
NR⁴⁴S(O)₂R⁴⁵group);
R³⁶ is hydrogen, halogen or C₁₋₄ alkyl;
R^{9'}, R^{10'}, R^{12'}, R^{13'}, R⁴², R⁴³ and R⁴⁴ are, independently, hydrogen, C₁₋₆ alkyl or phenyl; and,
R^{15'} and R⁴⁵ are, independently, C₁₋₆ alkyl or phenyl.

12. A compound as claimed in claim 11 wherein R¹ is phenyl substituted with one or more of fluorine, chlorine, C₁₋₄ alkyl or C₁₋₄ alkoxy.

13. A compound as claimed in claim 12 wherein R¹ is phenyl substituted by one, two or three of fluoro, chloro, methyl or methoxy.

14. A compound as claimed in claim 11 wherein R¹ is 3,4-dichlorophenyl or 3,4-difluorophenyl.

15. A compound as claimed in claim 11 wherein R¹ is 3,4-dichlorophenyl.

## Patentansprüche

1. Verbindung der Formel (XIIIa): 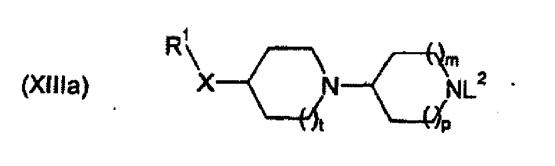 worin:
L² für Wasserstoff, *tert*-Butoxycarbonyl oder Benzyl steht;
t für 1 steht;
m und p für 1 stehen;
X für 0 steht;
R¹ entweder für ein- oder mehrfach durch Fluor, Chlor, C₁₋₄-Alkyl oder C₁₋₄-Alkoxy substituiertes Phenyl oder durch R³⁵ und R³⁶ substituiertes Phenyl steht;
R³⁵ für Wasserstoff, Cyano, S (O)₂-C₁₋₄-Alkyl, S (O) ₂-C₁₋₄-Halogenalkyl, Halogen, C₁₋₄-Alkyl, C₁₋₄-Halogenalkyl, C₁₋₄-Alkoxy oder Phenyl (gegebenenfalls substituiert durch ein oder zwei Halogenatome oder eine C (O) NR^{12'}R^{13'}-, NR^{9'} C (O) R^{10'} -, S (O) ₂R^{15'}-, S (O)₂NR⁴²R⁴³- oder NR⁴⁴S (O)₂R⁴⁵ -Gruppe) steht;
R³⁶ für Wasserstoff, Halogen oder C₁₋₄-Alkyl steht;
R^{9'}, R^{10'} , R^{12'}, R^{13'}, R⁴², R⁴³ und R⁴⁴ unabhängig voneinander für Wasserstoff, C₁₋₆-Alkyl oder Phenyl stehen und
R^{15'} und R⁴⁵ unabhängig voneinander für C₁₋₆-Alkyl oder Phenyl stehen.

2. Verbindung nach Anspruch 1, in der R¹ für ein- oder mehrfach durch Fluor, Chlor, C₁₋₄Alkyl oder C₁₋₄-Alkoxy substituiertes Phenyl steht.

3. Verbindung nach Anspruch 1 oder 2, in der R¹ für ein-, zwei- oder dreifach durch Fluor, Chlor, Methyl oder Methoxy substituiertes Phenyl steht.

4. Verbindung nach Anspruch 1, in der R¹ für 3,4-Dichlorphenyl oder 3,4-Difluorphenyl steht.

5. Verbindung nach Anspruch 1, in der R¹ für 3,4-Dichlorphenyl steht.

6. Verbindung der Formel (XIII): 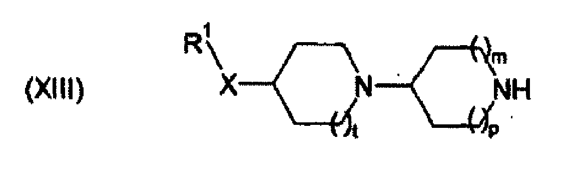 worin:
t für 1 steht;
m und p für 1 stehen;
X für 0 steht;
R¹ entweder für ein- oder mehrfach durch Fluor, Chlor, C₁₋₄-Alkyl oder C₁₋₄-Alkoxy substituiertes Phenyl oder durch R³⁵ und R³⁶ substituiertes Phenyl steht;
R³⁵ für Wasserstoff, Cyano, S (O) ₂-C₁₋₄-Alkyl, S (O) ₂-C₁₋₄-Halogenalkyl, Halogen, C₁₋₄-Alkyl, C₁₋₄-Halogenalkyl, C₁₋₄-Alkoxy oder Phenyl (gegebenenfalls substituiert durch ein oder zwei Halogenatome oder eine C (O) NR^{12'}R^{13'}-, NR^{9'} C (O) R¹⁰' -, S (O)₂R¹⁵'-, S(O)₂NR⁴²R⁴³- oder NR⁴⁴S (O)₂R⁴⁵ -Gruppe) steht;
R³⁶ für Wasserstoff, Halogen oder C₁₋₄-Alkyl steht;
R^{9'}, R^{10'}, R¹²', R^{13'}, R4², R⁴³ und R⁴⁴ unabhängig voneinander für Wasserstoff, C₁₋₆-Alkyl oder Phenyl stehen und
R¹⁵' und R⁴⁵ unabhängig voneinander für C₁₋₆-Alkyl oder Phenyl stehen.

7. Verbindung nach Anspruch 6, in der R¹ für ein- oder mehrfach durch Fluor, Chlor, C₁₋₄-Alkyl oder C₁₋₄-Alkoxy substituiertes Phenyl steht.

8. Verbindung nach Anspruch 7, in der R¹ für ein-, zwei- oder dreifach durch Fluor, Chlor, Methyl oder Methoxy substituiertes Phenyl steht.

9. Verbindung nach Anspruch 6, in der R¹ für 3,4-Dichlorphenyl oder 3,4-Difluorphenyl steht.

10. Verbindung nach Anspruch 6, in der R¹ für 3,4-Dichlorphenyl steht.

11. Verbindung der Formel (XIV): 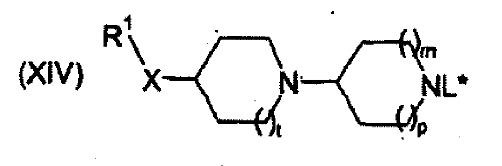 worin:
L* für BOC oder eine Benzylgruppe steht;
t für 1 steht;
m und p für 1 stehen;
X für 0 steht;
R¹ entweder für ein- oder mehrfach durch Fluor, Chlor, C₁₋₄-Alkyl oder C₁₋₄-Alkoxy substituiertes Phenyl oder durch R³⁵ und R³⁶ substituiertes Phenyl steht;
R³⁵ für Wasserstoff, Cyano, S (O) ₂-C₁₋₄-Alkyl, S (O) ₂-C₁₋₄-Halogenalkyl, Halogen, C₁₋₄-Alkyl, C₁₋₄-Halogenalkyl, C₁₋₄-Alkoxy oder Phenyl (gegebenenfalls substituiert durch ein oder zwei Halogenatome oder eine C(O)NR^{12'}R^{13'}-, NR^{9'}C(O)R^{10'}-, S (O)₂R^{15'}-, S(O)₂NR⁴²R⁴³- oder NR⁴⁴S (O)₂R⁴⁵-Gruppe) steht;
R³⁶ für Wasserstoff, Halogen oder C₁₋₄-Alkyl steht;
R^{9'}, R^{10'}, R^{12'}, R^{13'}, R⁴², R⁴³ und R⁴⁴ unabhängig voneinander für Wasserstoff, C₁₋₆-Alkyl oder Phenyl stehen und
R^{15'} und R⁴⁵ unabhängig voneinander für C₁₋₆-Alkyl oder Phenyl stehen.

12. Verbindung nach Anspruch 11, in der R¹ für ein- oder mehrfach durch Fluor, Chlor, C₁₋₄-Alkyl oder C₁₋₄-Alkoxy substituiertes Phenyl steht.

13. Verbindung nach Anspruch 12, in der R¹ für ein-, zwei- oder dreifach durch Fluor, Chlor, Methyl oder Methoxy substituiertes Phenyl steht.

14. Verbindung nach Anspruch 11, in der R¹ für 3,4-Dichlorphenyl oder 3,4-Difluorphenyl steht.

15. Verbindung nach Anspruch 11, in der R¹ für 3,4-Dichlorphenyl steht.

## Revendications

1. Composé de formule (XIIIa) : 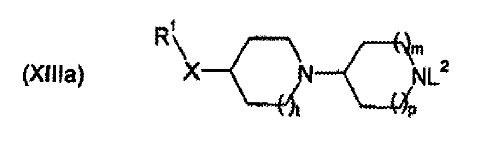 dans laquelle :
L² est un atome d'hydrogène, un groupe tert-butoxy-carbonyle ou un groupe benzyle ;
t vaut 1 ;
m et p valent 1 ;
X est un atome d'oxygène ;
R¹ est soit un groupe phényle substitué par un ou plusieurs parmi un atome de fluor, un atome de chlore, un groupe alkyle en C₁₋₄ ou un groupe alcoxy en C₁₋₄, soit R¹ est un groupe phényle substitué par R³⁵ et R³⁶ ;
R³⁵ est un atome d'hydrogène, un groupe cyano, un groupe S(O)₂(C₁₋₄-alkyle), un groupe S (O)₂(C₁₋₄-halogénoalkyle), un atome d'halogène, un groupe alkyle en C₁₋₄, un groupe halogénoalkyle en C₁₋₄, un groupe alcoxy en C₁₋₄ ou un groupe phényle (éventuellement substitué par un ou deux atomes d'halogène ou par un groupe C(O)NR^{12'}R^{13'}, un groupe NR^{9'}C(O)R^{10'}, un groupe S (O)₂R^{15'}, un groupe S(O)₂NR⁴²R⁴³ ou un groupe NR⁴⁴S(O)₂R⁴⁵) ;
R³⁶ est un atome d'hydrogène, un atome d'halogène ou un groupe alkyle en C₁₋₄ ;
R^{9'}, R^{10'}, R^{12'}, R^{13'}, R⁴², R⁴³ et R⁴⁴ sont, indépendamment, un atome d'hydrogène, un groupe alkyle en C₁₋₆ ou un groupe phényle ; et
R^{15'} et R⁴⁵ sont, indépendamment, un groupe alkyle en C₁₋₆ ou un groupe phényle.

2. Composé selon la revendication 1, dans lequel R¹ est un groupe phényle substitué par un ou plusieurs parmi un atome de fluor, un atome de chlore, un groupe alkyle en C₁₋₄ ou un groupe alcoxy en C₁₋₄.

3. Composé selon la revendication 1 ou 2, dans lequel R¹ est un groupe phényle substitué par un, deux ou trois parmi : un groupe fluoro, un groupe chloro, un groupe méthyle ou un groupe méthoxy.

4. Composé selon la revendication 1, dans lequel R¹ est un groupe 3,4-dichlorophényle ou un groupe 3,4-difluorophényle.

5. Composé selon la revendication 1, dans lequel R¹ est un groupe 3,4-dichlorophényle.

6. Composé de formule (XIII) : 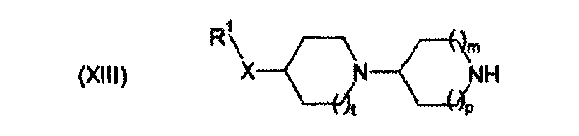 dans laquelle :
t vaut 1 ;
m et p valent 1 ;
X est un atome d'oxygène ;
R¹ est soit un groupe phényle substitué par un ou plusieurs parmi un atome de fluor, un atome de chlore, un groupe alkyle en C₁₋₄ ou un groupe alcoxy en C₁₋₄, soit R¹ est un groupe phényle substitué par R³⁵ et R³⁶ ;
R³⁵ est un atome d'hydrogène, un groupe cyano, un groupe S(O)₂(C₁₋₄-alkyle), un groupe S (O)₂(C₁₋₄-halogénoalkyle), un atome d'halogène, un groupe alkyle en C₁₋₄, un groupe halogénoalkyle en C₁₋₄, un groupe alcoxy en C₁₋₄ ou un groupe phényle (éventuellement substitué par un ou deux atomes d'halogène ou par un groupe C(O)NR^{12'}R^{13'}, un groupe NR^{9'}C(O)R^{10'}, un groupe S(O)₂R^{15'}, un groupe S(O)₂NR⁴²R⁴³ ou un groupe NR⁴⁴S(O)₂R⁴⁵) ;
R³⁶ est un atome d'hydrogène, un atome d'halogène ou un groupe alkyle en C₁₋₄ ;
R^{9'}, R^{10'}, R^{12'}, R^{13'}, R⁴², R⁴³ et R⁴⁴ sont, indépendamment, un atome d'hydrogène, un groupe alkyle en C₁₋₆ ou un groupe phényle ; et
R^{15'} et R⁴⁵ sont, indépendamment, un groupe alkyle en C₁₋₆ ou un groupe phényle.

7. Composé selon la revendication 6, dans lequel R¹ est un groupe phényle substitué par un ou plusieurs parmi un atome de fluor, un atome de chlore, un groupe alkyle en C₁₋₄ ou un groupe alcoxy en C₁₋₄.

8. Composé selon la revendication 7, dans lequel R¹ est un groupe phényle substitué par un, deux ou trois parmi : un groupe fluoro, un groupe chloro, un groupe méthyle ou un groupe méthoxy.

9. Composé selon la revendication 6, dans lequel R¹ est un groupe 3,4-dichlorophényle ou un groupe 3,4-difluorophényle.

10. Composé selon la revendication 6, dans lequel R¹ est un groupe 3,4-dichlorophényle.

11. Composé de formule (XIV) : 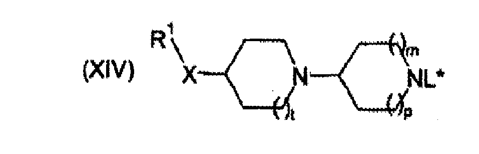 dans laquelle :
L* est un groupe BOC ou un groupe benzyle ;
t vaut 1 ;
m et p valent 1 ;
X est un atome d'oxygène ;
R¹ est soit un groupe phényle substitué par un ou plusieurs parmi un atome de fluor, un atome de chlore, un groupe alkyle en C₁₋₄ ou un groupe alcoxy en C₁₋₄, soit R¹ est un groupe phényle substitué par R³⁵ et R³⁶ ;
R³⁵ est un atome d'hydrogène, un groupe cyano, un groupe S(O)₂(C₁₋₄-alkyle), un groupe S (O)₂(C₁₋₄-halogénoalkyle), un atome d'halogène, un groupe alkyle en C₁₋₄, un groupe halogénoalkyle en C₁₋₄, un groupe alcoxy en C₁₋₄ ou un groupe phényle (éventuellement substitué par un ou deux atomes d'halogène ou par un groupe C(O)NR^{12'}R^{13'}, un groupe NR^{9'}C(O)R^{10'}, un groupe S(O)₂R^{15'}, un groupe S(O)₂NR⁴²R⁴³ ou un groupe NR⁴⁴S(O)₂R⁴⁵) ;
R³⁶ est un atome d'hydrogène, un atome d'halogène ou un groupe alkyle en C₁₋₄ ;
R^{9'}, R^{10'}, R^{12'}, R^{13'}, R⁴², R⁴³ et R⁴⁴ sont, indépendamment, un atome d'hydrogène, un groupe alkyle en C₁₋₆ ou un groupe phényle ; et
R^{15'} et R⁴⁵ sont, indépendamment, un groupe alkyle en C₁₋₆ ou un groupe phényle.

12. Composé selon la revendication 11, dans lequel R¹ est un groupe phényle substitué par un ou plusieurs parmi un atome de fluor, un atome de chlore, un groupe alkyle en C₁₋₄ ou un groupe alcoxy en C₁₋₄.

13. Composé selon la revendication 12, dans lequel R¹ est un groupe phényle substitué par un, deux ou trois parmi : un groupe fluoro, un groupe chloro, un groupe méthyle ou un groupe méthoxy.

14. Composé selon la revendication 11, dans lequel R¹ est un groupe 3,4-dichlorophényle ou un groupe 3,4-difluorophényle.

15. Composé selon la revendication 11, dans lequel R¹ est un groupe 3,4-dichlorophényle.
